Europäisches Patentamt

European Patent Office

Office européen des brevets

(11) Publication number: 0 261 872

A2

# (12) EUROPEAN PATENT APPLICATION

(21) Application number: 87308216.8

(51) Int. Cl.4: C12M 1/00

(22) Date of filing: 17.09.87

(30) Priority: 23.09.86 GB 8622819
28.07.87 GB 8717831

(43) Date of publication of application:
30.03.88 Bulletin 88/13

(84) Designated Contracting States:
AT BE CH DE ES FR GB GR IT LI NL

(71) Applicant: BIOTECHNA LIMITED
81 Wimpole Street
London WIM 7DB(GB)

(72) Inventor: Robinson, Lee Fisher
3, Athenaeum Hall
Vale of Health London NW3 1AP(GB)
Inventor: Morrison, Angus William
East Stansted Tilburstow Hill
South Godstone Surrey RN9 8NA(GB)
Inventor: Bamforth, Mark Robert
38, Stevenage Road
Hitchin Hertfordshire SG4 9DR(GB)

(74) Representative: BATCHELLOR, KIRK & EYLES
2 Pear Tree Court Farringdon Road
London EC1R 0DS(GB)

(54) Improvements relating to biosynthesis.

(57) A biosynthetic process in which a synthesis mix-
ture is caused to flow under photophobic conditions
through a substantially opaque tube wound on an
upstanding core structure and one or more synthesis
products are withdrawn from the mixture.

FIG.I.

## Improvements Relating to Biosynthesis

This invention relates to biosynthesis and is particularly concerned with a bioreactor and a method of biosynthesis employing such a bioreactor.

Biosynthetic routes involving biomass culture for obtaining commercially valuable products have been in use for a considerable time. Many examples occur in the brewing industry, for example in yeast fermentations and the pharmaceutical industry, for example in the preparation of penicillin and cephalosporin antibiotics.

There is a constant need for improvements in carrying out such biosynthesis. Thus, for example, fermentation reactions are conventionally carried out in large scale fermenters in which it is difficult to control the reaction conditions, for example the degree of agitation required for optimum yield. Such fermenters are also difficult to clean.

Other problems encountered include the growth of unwanted microscopic species, such as bacteria, amoeba and rotifers within the biomass culture.

The present invention seeks to improve an improved biosynthesis process which can be carried out on a substantial commercial scale using organisms which flourish in the absence or partial absence of light.

According to a first aspect of the present invention a biosynthetic process comprises causing a synthesis mixture comprising living plant matter, capable of growth in the absence or partial absence of light, together with the essential nutrients for growth of the plant matter, to flow through a substantially opaque tube wound on an upstanding core structure and withdrawing one or more synthesis products from the mixture.

Preferably the substantially opaque tube forms part of a closed circuit through which the synthesis mixture is continuously caused to flow with the synthesis products being continuously withdrawn from the mixture and the nutrients and make-up liquor being continuously added thereto.

In one method of operating such a closed circuit, a quantity of the fluid is withdrawn from the circuit, solid biomass product is filtered from the fluid, nutrient make-up liquor is added to the filtrate and, after being sterilised, the filtrate is returned to the closed circuit.

In this way, the withdrawal of the biomass product from the fluid and the addition of nutrient liquor to the filtrate takes place outside the closed circuit and the filtrate is sterilised before it is returned to the closed circuit. Thus, the sterile conditions in the closed circuit are not lost.

After the filtrate has been sterilised and cooled, carbon dioxide may be introduces into it prior to it being returned to the closed circuit.

According to a second aspect of the invention a bioreactor comprises an upstanding core structure, a substantially opaque tube wound on the core structure, means for causing a synthesis mixture comprising living plant matter, capable of growth in the absence or partial absence of light, together with essential nutrients for growth of the plant matter to flow through the wound tube and means for withdrawing one or more synthesis products from the mixture.

Conveniently the tube is of polyethylene which is either opaque or is provided with an opaque coating.

The core structure may be of substantially cylindrical form with the tube wound helically on the cylinder, The tube may be in the form of a single helical layer on the core or a number of layers may be provided. Alternatively, the tube may be wound as a series of concentrixc coild plate structures connected in a vertical stack.

Preferably the synthesis mixture is pumped to the top of the core structure and allowed to flow downwardly through the wound tube under turbulent conditions. Alternatively, the synthesis mixture may be pumped through the tube to a header tank, with suitable gases introduced at the base of the tube.

The method may be carried out under aerobic an anaerobic conditions. Thus carbon dioxide or air may be supplied to the tube or other gases such as oxygen or air/oxygen mixtures may be employed dependent on the synthesis produce desired. Some biosynthetic reactions proceed anaerobically, in which case no such gaseous imput is required.

In one preferred form of the invention, the fact that some biosynthetic reactions proceed aerobically while some proceed anaerobically, can be utilised by providing two or more reactors, as described above, operating in series, a first reactor (or bank of reactors) being used to carry out an anaerobic reaction which leads to evolution of gas such as carbon dioxide, which, after separation of the first product biomass, is used in a second reactor for an aerobic reaction utilising the gas.

According to a further preferred form of the invention, ammonia gas is used as the, or as one of the nitrogen sources. The use of controlled ammonia injection has been found to be beneficial in minimising the growth of unwanted microscopic species, such as bacteria, amoeba and rotifers. It is believed that the presence of ammonium salts and ammonium ions inhibits such growth, while acting as a nutrient source for the growth of the plant material.

The nutrients for the synthesis may be provided at least in part by waste effluents such as those from sugar plants or petroleum refinery wastes or other high BOD carbohydrate wastes, the wastes thus being purified in the process, so that the biomass produced in a valuable byproduct of the effluent treatment process.

A particularly preferred tube material is polyethylene, especially low density polyethylene, which is low cost and also has the valuable advantage of being resistant to attack by the biomass medium. The tubing is suitably of black or dark opaque coloration or is given an opaque lining or is coated with a paint to give such a finish. Metallic tubes may also be employed. If desired the opaque tubing may be exposed to sunlight to absorb heat for the reaction. In this case the tubing may be given a protective coating to inhibit sunlight deterioration. If desired the tubing may be of a reinforced type or encased in resin for strengthening purposes, e.g. for pressure reactions.

The invention will now be described by way of example with reference to the accompanying drawings wherein:

Figure 1 is a diagrammatic view of a bioreactor in accordance with the invention;

Figure 2 is a sketch illustrating the layout of a plurality of such reactors;

Figure 3 is a flow diagram of two banks of bioreactors operating in series.

Figure 4 is a diagrammatic view of an alternative form of reactor;

Figure 5 illustrates a process of aerobic and anaerobic stages; and

Figure 6 is a flow diagram of a closed system for biomass culture.

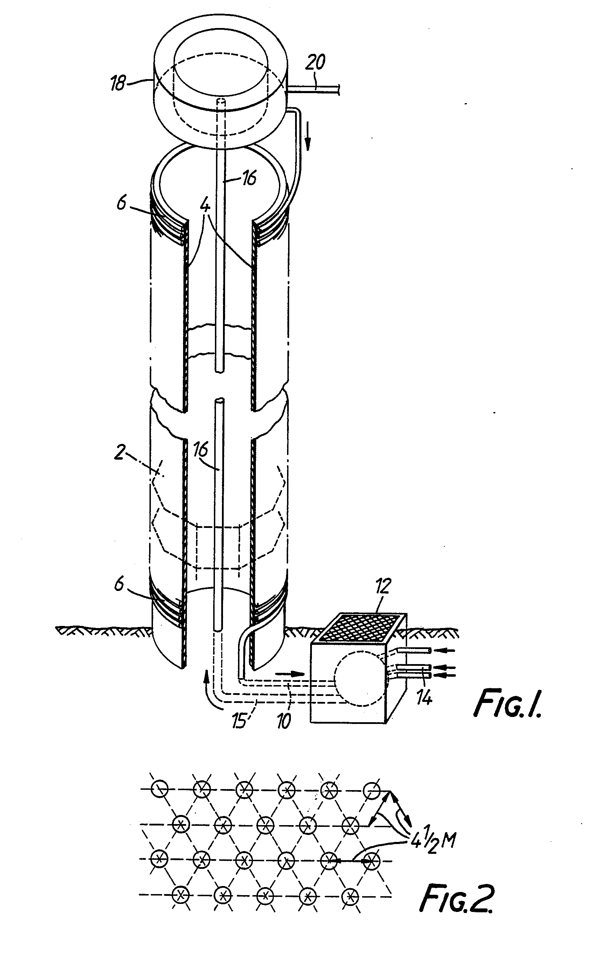

The bioreactor shown in Figure 1 comprises a core structure 2 (shown in dotted lines) which is upstanding and substantially cylindrical. The core structure may provide a continuous outer surface and be formed for example of hollow concrete sections. Dependent on the size of the reactor, the concrete sections may have internal access steps to enable inspection and maintenance personnel to enter the interior and reach apparatus positioned at the top part of the core structure. Alternatively, the core structure may be of openwork construction, for example it may be constructed of metal supports such as those known under the trademark "Dexion", or alternatively the core may be of cylindrical shaped metal mesh construction.

Over the core structure 2 is a tube 6 of substantially opaque material wound in a flat spiral. A preferred material is polyethylene, preferably of low density, as such tubing has low cost, and can be readily extruded and wound in long lengths. Polyethylene also has the advantage of good corrosion resistance and can withstand the chemical conditions of the reaction mixture to a much greater extent than a material such as stainless steel, which has conventionally been used in bulk fermentation methods. However, other plastics materials, such as opaque plasticised polyvinyl chloride, can be used, or rubber. Materials, such as darkened glass, if capable of withstanding the conditions of use, can alternatively be used. Pegs (not shown) may project from the core structure 2 to support the tube and prevent slippage of the windings.

If desired for reasons of strength, the tubing may have a reinforcing outer coating, for example of resin reinforced with glass fibres or other fibres. This is especially valuable if it is desired to carry out the biosynthesis under considerable pressure.

The core structure may be solid, for exemple an existing tank on which the tube windings are arranged. The tanks may be of any desired diameter, e.g. from 2 to 5 meters.

The lower end of the core structure 2 is mounted in the ground and the lower end 10 of the tube 5 extends through the ground to a control pumping device generally indicated at 12. Pumping device 12, which can contain a diaphragm pump or any other suitable type of pump, is connected to tube 6, central tube 16 and supply lines 14 are, for example carbon dioxide and/or air, nutrients and a nitrogen source, such as that provided by ammonia, ammonium salts, urea, compound fertilizers, etc., to be added to the flow of synthesis mixture. The supply lines may conveniently be computer controlled. An outlet tube 15 carries the synthesis mixture from pumping device 12 up a central tube 16 arranged to extend up the hollow center of core structure 2 to a header tank 18. Header tank 18 can contain any suitable launder means (not shown) to enable a product stream to be withdrawn on line 20. For example, more concentrated product rising towards the top of the mixture in the launder may be withdrawn by means of a weir device. Alternatively other separation means, such as a hydrocyclone, can replace the header tank 18. Line 20 is shown as extending from the side of header tank 18; however it may equally well extend down the centre of core structure 2 so that product is withdrawn at the base of the structure. The header tank also contains a purge system to remove gases produced.

It will be appreciated that alternatively the synthesis mixture may be pumped up the coild tube to the header tank 18, with suitable gases introduced at the base of the coild tube, the return line 16 from the tank 18 to the pump having, if required, a flowmeter and/or a heat exchanger or like devices incorporated therein.

The dimensions of the bioreactor will vary dependent on the biomass production being carried out.

Examples of use of the bioreactor are the fermentation of yeasts and the cultivation of moulds, for example to give cephalosporin or penicillin antibiotics.

While it will be appreciated that tube diameter can vary, the diameter of the tubing should remain sufficiently small to encourage turbulence as the material passes through the coil. Turbulence assists in preventing unwanted coating of the inside of the tube, thus increasing the working life of the plant. Preferably the turbulence is such as to give a Reynolds number of at least 2000.

The bioreactor shown has its pumping device 12 buried in the ground so as to maximise the space between each bioreactor, although of course the pumping device could be above ground level. In the example shown in Figure 2 it will be seen that burying the pumping devices gives adequate spacing to allow the passage of servicing and product collection vehicles between the bioreactors. Such servicing may include provision for coping with extreme weather conditions, for example a heat exchanger included in the circuit and for example, in cold overnight conditions with the possibility of frost, it is possible to install removable insulated jackets positioned over the bioreactors when necessary. Likewise, in very hot conditions coolant can be sprayed on the reactors from external service devices. Alternatively, the bioreactors themselves may include provision for directing coolant spray onto the tubing 6 when desired, such as the provision of a water spray ring at the top of core structure 2 fed by a supply tube passing up the hollow core structure. Of course, the bioreactors need not operate in an external environment.

In a modification (not shown) of the reactor shown, the core structure 2 is mounted on a rotating platform with means provided for the product withdrawal via a central circular launder leading to a tube extending to the base of the core structure. Such an arrangement has the advantage of using the rotational speed to influence flow characteristics in the tube.

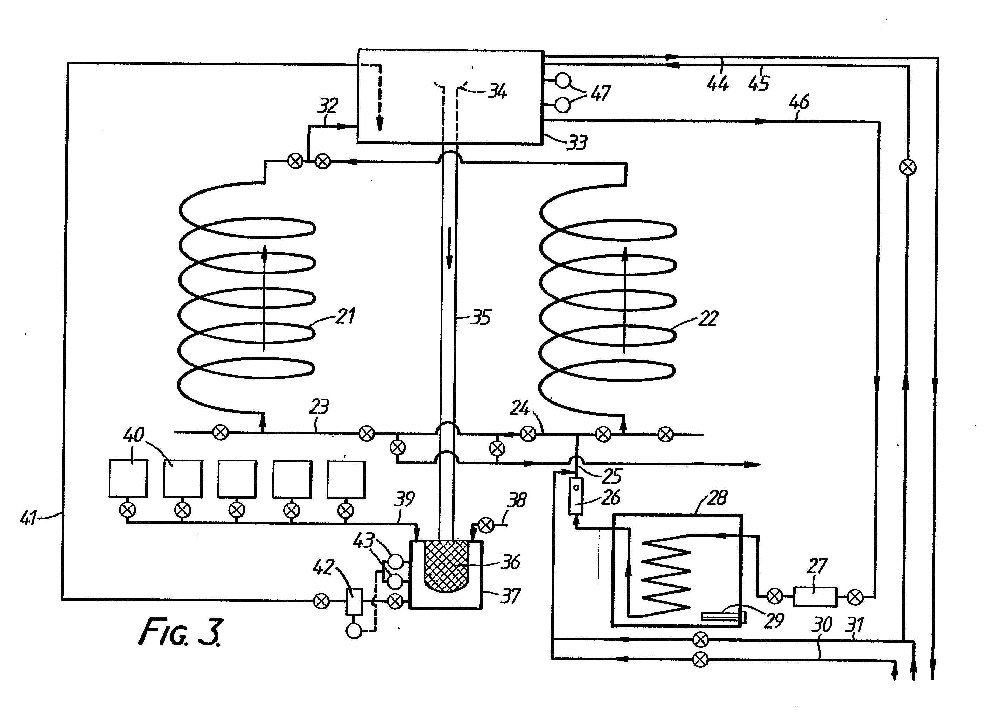

An alternative method of biomass production suitable for use on a large scale uses the plant illustrated in Figure 3. The plant comprises a pair of substantially opaque coiled tubes 21 and 22 arranged in parallel and each mounted on a suitable core structure (not shown). The coils may for example be plastics tubing of 500 metres length and 30 mm diameter. It will be appreciated that, although two coils are shown, further coils may be employed. The synthesis mixture is fed to the coils 21 and 22 via lines 23 and 24 from a single line 25 which is supplied via flowmeter 26 with recirculating reactants pumped by pump device 27 (suitably a diaphragm pump). Before reaching line 25, the recirculating reactants pass through heat exchange unit 28 provided with immersion heater 29. Unit 28 serves to provide heat if necessary in cold weather, or alternatively to remove heat during hot ambient conditions. Carbon dioxide and air are also supplied to line 25 via suitable flowmeters (not shown) on lines 30 and 31 respectively.

At the tops of the coils 21 and 22, the flowing synthesis mixtures join in line 32 for passage to header tank 33 with an overflow harvester device 34 which allows mixture to flow downwardly through return pipe 35 to a bag filter 36 in which product biomass is collected and periodically withdrawn. The bag is of a mesh which retains large mature product while allowing smaller growing product to pass through. Bag filter 36 is disposed in filter unit 37 supplied with make-up water on line 38 and nutrients on line 39 from a series of nutrient supply vessels 40. Filtrate in unit 37 with added water and nutrients is returned on line 41 to header tank 33 under the action of pump 42 which is responsive to level control valves 43.

Excess carbon dioxide (and/or other gaseous products) are withdrawn from header tank 33 on line 44. When necessary air is supplied to header tank 33 on air purge line 45. Circulating syntheses mixture not passing down overflow 34 returns to pump 27 on line 46.

Circulation in the system and harvesting of product are controlled by electronically operated product control valves 47, which are brought in operation after start up once optimum synthesis conditions have been achieved (as determined for example by optical density measurement).

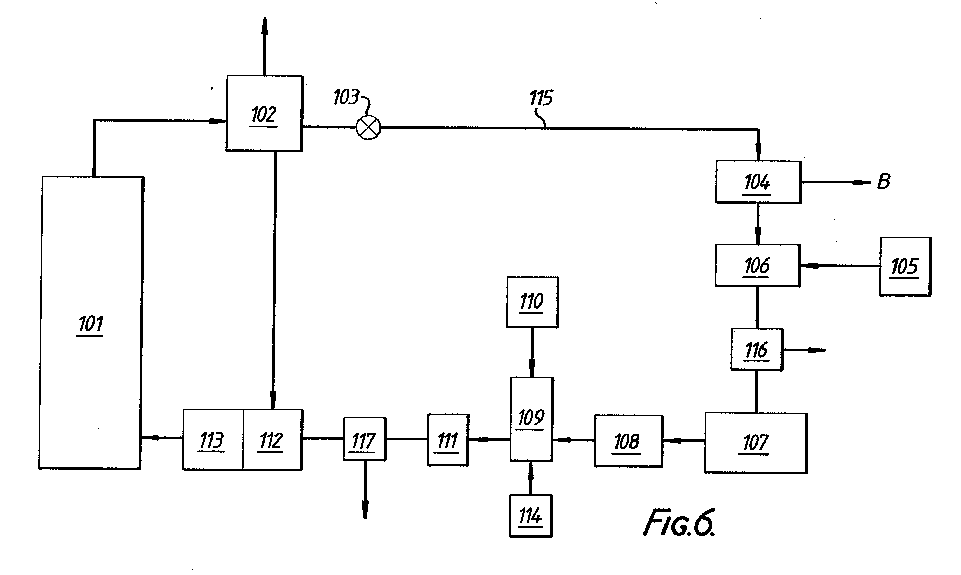

In the circulation system a bleed-off recovery and sterilisation circuit may be incorporated, for example, a circuit as described further below in relation to Figure 6.

It will be noted that in the apparatus described above, the synthesis mixture is pumped up the coils and product flows downwardly under gravity through return pipe 35. The straight and comparatively long down return pipe 35 to the filter bag 36 gives a high velocity which prevents clogging.

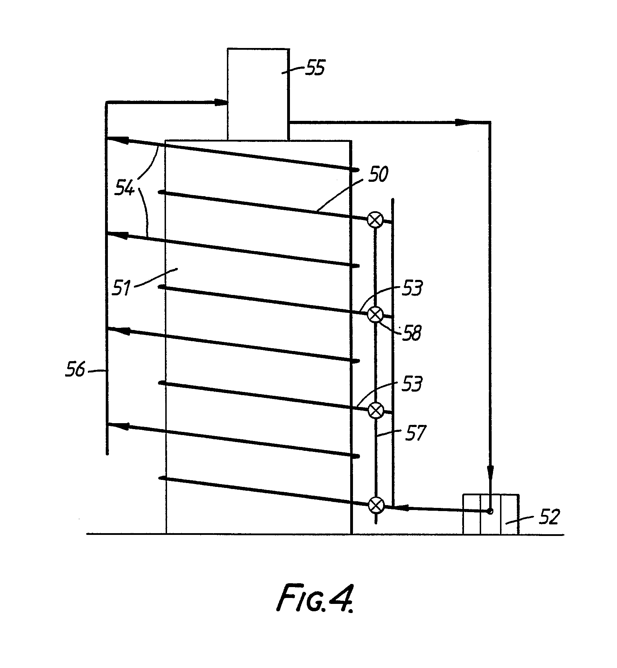

When using tubular modules, provision may be made for intermediate pumps, and/or air or steam injection so as to control the flow rates through the tubing even for long flow passages. This is particularly valuable when the reaction medium has a tendency to become viscous, for example in certain fermentation processes. Figure 4 schematically illustrates a modified reactor which embodies provisions to overcome problems of pressure drop (and also possible elongation of the tubing when warm, e.g. at 35°C). Thus a coil 50 is arranged in sections on core 51. Synthesis mixture is pumped up the coil via the recycle pump and heat exchanger unit 52 but, instead of being all introduced at the bottom of the coil, it is introduced therealong on a series on inlet lines 53. Necessary reaction gases are also introduced to each line 53 via a feed main 57 having valve entries 58 to each line 53. Similarly outlet lines 54 lead from each coil section to header tank 55 via rising main 56. It is advantageous to construct rising main 56 so that it can move on the side of the coil to cater for expansion of the tubing. Thus the main may be flexible or may be rigid but provided with a flexible mounting. The lengths of tube in each coil section will vary according to flow rate, tubing bore and tower height etc. but approximately eight sub units with lengths of about 300 metres and 20 mm tubing bore have been found to be suitable.

It will be appreciated that the bioreactors are easy to assemble and if wished can be constructed in modular form. Thus the tubular coil can readily be constructed in sections with valves and junctions allowing reaction products to be removed as needed and/or any necessary additional nutrient feed introduced. This is especially useful for rapid reactions, such as certain fermentation reactions.

Although pump operation has been described above for providing the motive force necessary to give highly turbulent flow in the tubing, as is often desirable, in some reactions it is desirable to employ lower flow conditions, for example, where the product cells are of a delicate nature. In such cases it may be sufficient to employ air and/or other gaseous supply as the lift to sustain circulation. If need be a compressed gas venturi jet or a steam jet may be employed. Steam injection is particularly suitable where a certain amount of heat is required for growth.

The method and apparatus described above are applicable to a wide range of biomass production processes. If desired, the feed system to the reactor can be controlled to introduce small amounts of one or more trace elements such as selenium, cobalt, copper, zinc, gallium and germanium under varying conditions to alter trace element amounts. It will be seen that the process allows the production of single strain pure biomass under controlled conditions to give a pure and consistent product. Dependent on the ancillary processing employed, the concentration of certain valuable products in the biomass can be increased.

It will be appreciated that considerable variation is possible in the nutrients supplied to the bioreactor and the operating conditions.

It has been found of value to use ammonia gas as one of the nitrogen sources, or as the sole nitrogen source. Controlled injection of ammonia gas, while promoting the growth of certain organisms, inhibits the growth of unwanted microscopic life such as bacteria, amoeba and rotifers. This is of considerable commercial importance as the growth of such microscopic species has been a major problem in previously proposed methods for biomass production. Other gases which inhibit bacterial growth, such as carbon monoxide, may be employed.

As an alternative, or in addition, bacterial invasion problems may be at least partially alleviated by the use of ultraviolet irradiation of transparent sections of the tubing. This may, for example, be accomplished by winding an ultraviolet emitting coil around one or more transparent portions of the tube and/or by the inclusion of an ultraviolet emitting tube within the tube itself, suitably in a specially widened tube section.

The reactor described above is suitable for use for both aerobic and anaerobic biomass production processes. Thus gases such as carbon dioxide or air may be used, for example for Spirulina and Chlorella production, while air/oxygen mixtures or oxygen alone may be employed for certain processes such as yeast growth. Anaerobic processes may also be carried out.

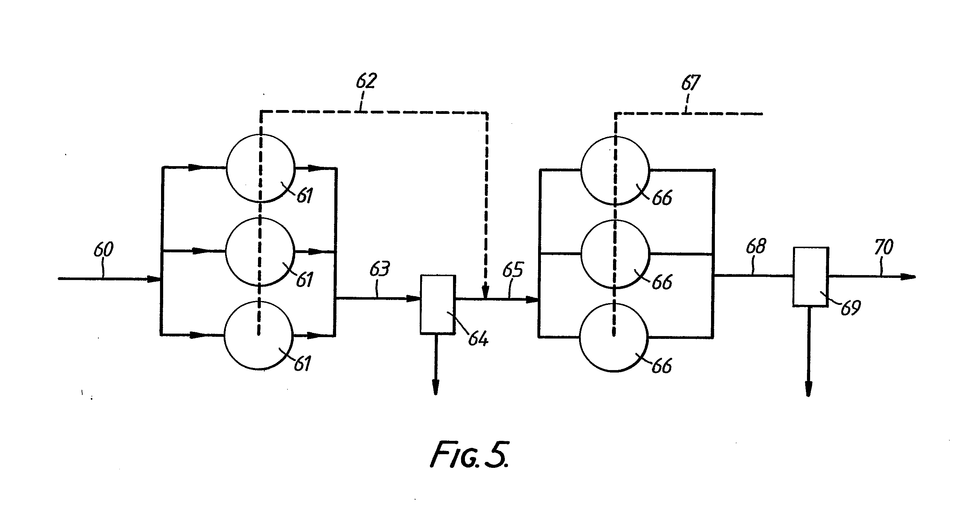

According to a useful further embodiment of the invention, two bioreactors, or banks of bioreactors, may be joined in series, the first being used for an anaerobic reaction while the second reactor utilises the $CO_2$ in the cultivation of $CO_2$-utilising, oxygen-producing algae. Such a system is illustrated in Figure 5.

In the flow diagram of Figure 5, a high BOD effluent liquor is passed on line 60 to a first bank of reactors 61 as nutrient for the cultivation of a carbon dioxide-producing organism. Carbon dioxide produced in reactors 61 is withdrawn on line 62. Liquor from reactors 61 is withdrawn in line 63 to filter unit 64, where solid biomass is withdrawn. The carbon dioxide stream in line 62 is combined with the liquor from filter 64 in line 65 for passage as nutrient to a second bank of reactors 66 for the cultivation of a carbon dioxide-consuming, oxygen-producing organism. Oxygen is withdrawn on line 67 for any suitable purpose, while the liquor withdrawn on line 68 is filtered in unit 69 to give a solid biomass product. The remaining effluent on line 70 has a considerably reduced BOD value. Both of the banks of reactors 61 and 66 comprise opaque tubing in accordance with the present invention for carrying out a bioreaction in the absence of light.

Of course further stages may be added and any desired number of bioreactors may be used in each bank. Any other gases produced in the first reactors may be used in subsequent bioreactors or for other purposes. The use of a plurality of reactors can also prove useful when one reactor is used for a photosynthetic reaction while the other reactor is used for a biosynthetic reaction proceeding in the absence of light.

The provision of a continuously operable process with recycling of the mixture keeps the consumption of gases and nutrients as low as possible with minimum wastage. Product gases can be used in any adjacent chemical plant. Any heat produced can be employed in heat exchange. High biomass densities can be obtained. Even derelict and uneven land can be utilised and the size of the installation can readily be expanded to fill growing needs. Only low grade heat at most is required even in a severe winter to keep a temperature minimum of approximately 10°C. Cooling in summer is desirable to maintain a suitable maximum temperature. The use of turbulent conditions enables long running periods before the need for cleaning, so that shut down periods are kept to a minimum. In any case cleaning of the tubing is a comparatively straightforward operation and can be effected by forcing a cleaning "bullet" through the tubing. This in contrast to the problems encountered in cleaning conventional fermentation tanks where contamination often has to be scraped from the walls.

It will be seen that the system described above lends itself to automatic control and the whole arrangement of modules can be computer controlled, thereby saving labour. A further advantage is that part of such a multi-module system can be switched, if required, from producing one type of product to another, without the need for major plant modification.

In Figure 6, numeral 101 indicates a reactor of the type shown in Figure 1 in which a biomass culture takes place under sterile conditions at a processing temperature of, say, 28 - 30°C. The fluid is continuously pumped from the container to a disengagement header 102 and back to the container 101 by way of a recycle pump 112 with a heat exchanger 113 if necessary. In the disengagement header 102 gas generated during the process is allowed to escape to atmosphere.

A valve 103 is located in a pipe 115 leading from the disengagement header 102 and, periodically, this valve is opened to allow a bleed-off of the fluid from the disengagement header 102. This fluid may be a constant bleed of, say, 5 - 10% of the circulating fluid in the closed system or the valve may operate at set intervals, say, hourly, to deliver 10 - 20% of the total fluid from the reactor 101. The fluid flowing along the pipe 115 enters a filtration system 104 from which the solid biomass product B, such as spirulina, is removed from the liquid filtrate. The filtration system can be one of any of a number of known types, i.e. rotary vacuum, microstrainer, edge filters or simple Buchner types or even filter presses. The particular form of filtration system is chosen for the particular type of biomass culture which is being produced. From the filtration system the filtrate is passed to a mixing tank 106 into which additional nutrients are added from a source 105. This nutrient is in the form of a liquor and is added to the filtrate in the tank according to an automatic analyser which indicates the levels of carbonate, phosphate, nitrate or other chemicals as appropriate in the filtrate.

As the sterile conditions in the reactor 101 may not be retained in the filtration system 114 and the mixing tank 106, the filtrate with the added nutrients are passed through a steriliser 107 which serves to kill off all hostile bacteria and other living substances contained within the filtrate. Prior to entering the steriliser, the filtrate is conveniently passed through an ultra filtration unit in the form of a micro-filter 116 to remove any remaining solids of at least 0.2 microns. The sterilisation system is usually a steam steriliser. Any residual biomass materials and killed bacteria and other materials may be filtered off from the sterilised liquid. From the steriliser the liquid is passed through a cooler 108 to reduce the temperature to approximately 20°C. If desired, the cooled liquid may then be introduced to an absorption system 109 where carbon dioxide from a source 110 is added in the appropriate quantity to react with the alkaline hydroxide present in the liquid to raise the carbonate and bicarbonate in the solution to the required level. The amount of carbon dioxide which is added can be automatically controlled by an analyzer operating through an electrically controlled relay system. This absorption unit 109 can be pressurised if desired to ensure $CO_2$ solution. Sterile innoculum may be introduced into the absorption unit 109 from a source 114. The treated liquor is then pumped by a dosing pump 111 into the inlet side of the recycle pump 112 and it is returned into the closed system of the reactor 101 and the disengagement header 102. A further ultra filtration unit in the form of a micro-filter 117 to remove any solids of at least 0.2 microns may be positioned between the dosing pump 111 and the recycle pump 112. A heat exchanger 113 may be positioned between the recycle pump 112 and the reactor 101 to ensure that the processing temperature is at the required level.

The advantage of the arrangement illustrated in Figure 6 is that the main photophobic system remains in sterile conditions and the production of wanted biomass is produced continuously in the sterile conditions. The biomass is removed by a bypass unit where the biomass is removed from the liquid and additional nutrient liquor is added to the liquid. The liquor is sterilised before it is returned to the closed system.

**Claims**

1. A biosynthetic process comprising causing a synthesis mixture comprising living plant matter, capable of growth in the absence or partial absence of light, together with essential nutrients for growth of the plant matter, to flow through a substantially opaque tube wound on an upstanding core structure and withdrawing one or more synthesis products from the mixture.

2. A process as claimed in claim 1 wherein the essential nutrients for growth comprise carbon dioxide and a source of nitrogen.

3. A process as claimed in claim 2 wherein ammonia gas is a source of nitrogen.

4. A process as claimed in any preceding claim in which the synthesis mixture is pumped to the top of the core structure and allowed to flow downwardly through the tube under turbulent conditions.

5. A process as claimed in claim 1, 2 or 3 wherein the synthesis mixture is pumped upwardly through the tube towards the top of the upstanding core structure.

6. A process as claimed in any preceding claim wherein an anaerobic synthesis is carried out in the tube which leads to evolution of gas and after separation of a biomass product the mixture is passed through a second substantially opaque tube in which an aerobic synthesis is carried out using at least some of the evolved gas and a biomass product is withdrawn from the second tube.

7. A biosynthetic process as claimed in claim 1 in which the substantially opaque tube forms part of a closed circuit through which the synthesis mixture is continuously caused to flow with the synthesis products being continuously withdrawn from the mixture and nutrients being continuously added thereto.

8. A process as claimed in claim 7 wherein a quantity of the fluid is withdrawn from the closed circuit, solid biomass product is filtered from the fluid, nutrient make-up liquor is added to the filtrate and, after being sterilised, the filtrate is returned to the closed circuit.

9. A bioreactor comprising an upstanding core structure, a substantially opaque tube wound on the core structure, means for causing a synthesis mixture to flow through the wound tube and means for withdrawing one or more synthesis products from the mixture.

10. A bioreactor as claimed in claim 9 in which the core structure is of generally cylindrical form and the tube is wound helically on the cylindrical core.

11. A bioreactor as claimed in claim 10 in which the tube is wound in a number of layers on the core.

12. A bioreactor as claimed in claim 9, 10 or 11 in which the tube is of polyethylene or other suitable plastics material or rubber which is either opaque or is provided with an opaque coating.

FIG.I.

FIG.2.

Fig. 3.

FIG.4.

FIG. 5.

0 261 872

FIG.6.

0 261 872